# EUROPEAN PATENT APPLICATION

(11) **EP 3 808 308 A1**
(43) Date of publication of application: **21.04.2021**
(21) Application number: 18922665.7
(22) Date of filing: 15.06.2018
(51) Int. Cl.: A61C 8/00, A61L 27/06, A61L 27/08, C23C 4/00, B33Y 10/00, B33Y 70/00, B33Y 80/00, B82Y 30/00

(54) **METHOD OF PRODUCING A DENTAL IMPLANT USING A COMPOSITE NANOCOATING**

(71) Applicant: Narayama Limited Liability Company, Moscow, 143026 (RU)
(72) Inventor: FADEEV, Ivan Anatolievich, Chelyabinsk, 454071 (RU); DIURIAGIN, Aleksei Sergeevich, Chelyabinsk, 454091 (RU); DIURIAGIN, Vasily Sergeevich, Chelyabinsk, 454091 (RU); ORLOV, Vasily Sergeevich, Chelyabinskaya obl., 456510 (RU); DENISOV, Alexei Vyacheslavovich, Chelyabinsk, 454031 (RU)
(74) Representative: Berggren Oy, Helsinki & Oulu
(86) International application number: PCT/RU2018/000397
(87) International publication number: WO 2019/240608

(57) **Abstract**

This invention relates to the field of medical technology, and can be used in dentistry and traumatology, in particular when creating dental implants. Namely, the invention relates to the development and creation of a method for producing a dental implant characterized by high strength, as well as increased ability to activate the process of osteogenesis and osseointegration. The implant obtained by this method is characterized by high biocompatibility, bactericidal properties (reduces pronounced dystrophic and necrotic processes of living tissue), and an increased level of implant surface strength.

## Description

### Technology area

This invention relates to the field of medical technology, and can be used in dentistry and traumatology, in particular when creating dental implants. The resulting dental implant with a composite coating promotes the activation of the osteogenesis process, has biocompatible and antibacterial properties, and has an increased level of consumer properties.

### State of the art

An implant is a man-made material or device that is surgically placed into the body. One of the urgent problems of traumatology, orthopedics and dentistry is the creation of new osteoplastic materials and the use of various implants for osteosynthesis. In recent years, titanium implants have been widely used, which make it possible to form a bioactive surface due to the content of calcium-phosphate compounds on the surface. The surface on the implants has a multilevel porous structure with a rough surface, which has adhesive and osteosynthetic properties. Biocompatibility and bioactivity of implants made of titanium or titanium alloys intended for use in various fields of medicine: dentistry, orthopedics, traumatology, is achieved due to the formation of a bioactive coating on their surface, similar to the composition of bone tissue and with a highly developed surface structure. Hydroxyapatite is a promising material for creating bioactive calcium-phosphate coatings that are applied to a base made of titanium and its alloys, the use of which improves the ability of the implant to integrate into the bone tissue of a living organism.

A known method of coating is the electrochemical method under spark discharge conditions [RU2154463]. The coating is applied during anodizing in a saturated solution of hydroxyapatite in phosphoric acid with a concentration of 5-20% or 3-5% of a suspension of hydroxyapatite with a dispersion of less than 100 microns in this saturated solution. The anodizing process is carried out with a pulsed or direct current under spark discharge conditions at voltages up to 150 V. As a result of using this method, a titanium-based bioactive coating is obtained, consisting of titanium oxide, calcium and phosphorus. However, it is not possible to create a bioactive layer on the surface of the implant with high strength.

A known method of coating an implant made of titanium and its alloys [RU 2221904], including anodizing the implant with a pulsed or direct current in a spark discharge with a pulse repetition rate of 0.5-10.0 Hz in solution phosphoric acid for 10-30 minutes with constant stirring, and anodizing is carried out at a voltage of 90-100 V and 20-35 ° C in a phosphoric acid solution with a concentration of 5-33%, containing CaO powder to a supersaturated state, or in a phosphoric acid solution with a concentration of 5-25%, containing CaO powder to a supersaturated state and an additional 5-10% suspension of hydroxyapatite with a dispersion of less than 70 microns to create a suspension.

There is also known a method of manufacturing an intraosseous dental implant with a plasma-sprayed multilayer bioactive coating [RU2146535], including the plasma spraying of a coating system of five layers of different fineness and thickness: the first two from titanium or titanium hydride, the next two layers from a mixture of titanium or titanium hydride with hydroxyapatite, differing in the content of components in the layers, and the outer, fifth layer of hydroxyapatite. Spraying is carried out layer by layer under various modes that provide a smooth transition from the compact structure of the titanium base of the implant through a multilayer transition coating system to a thin biologically active porous layer.

A known method of applying a calcium-phosphate coating on an implant made of titanium and titanium alloys, including anodizing the implant with a pulsed current in a spark discharge in a phosphoric acid solution containing hydroxyapatite, while anodizing is carried out with a pulsed current with the following parameters: pulse time 50-200 µs; pulse repetition rate 50-100 Hz; initial current density 0.2-0.25 A / mm2; the final voltage is 100-300 V, and the phosphoric acid solution additionally contains calcium carbonate [RU2291918].

A known method of manufacturing an intraosseous dental implant with carbon nanocoating [RU2571559] includes layer-by-layer plasma spraying on the implant base of biocompatible coatings made of solid amorphous diamond-like carbon. The coating consists of a large number of layers consisting of a compound of titanium and carbon, however, as a result of the studies carried out, it was revealed that this coating is unstable and prone to self-destruction due to high internal stress. Thus, this method does not provide sufficient mechanical strength of a multilayer coating on the surface of a metal implant, and due to mechanical damage to the coating, resorption of the coating and a decrease in its bactericidal properties take place.

Despite the large number of existing methods for creating an implant and applying a bioactive coating to their surface, there is still a need to develop and create an implant characterized by high strength and increased ability to activate osteogenesis processes.

### Disclosure of invention

The objective of this invention is to develop and create a dental implant characterized by high strength, as well as increased ability to activate the process of osteogenesis and osseointegration.

The technical result of this invention is the development and creation of a method for producing a dental implant with a bioactive nanocoating based on a calcium-phosphate-carbon composite, characterized by high strength, as well as increased ability to activate the process of osteogenesis and osseointegration. The implant obtained by this method is characterized by high biocompatibility, bactericidal properties (reduces pronounced dystrophic and necrotic processes of living tissue), and an increased level of implant surface strength.

The specified technical result is achieved by implementing a method for producing a dental implant with a bioactive nanocoating based on a calcium-phosphate-carbon composite, including the following steps:
a) printing the implant on a titanium 3D printer;
b) treatment of the surface of the implant obtained at stage a, including the treatment of the implant with argon ions accelerated to 1 keV at a pressure of (2-6) × 10-2 Pa with the application of a negative bias voltage with a gradual increase from 800 to 1500 V for 1 h;
c) layer-by-layer deposition on the implant base, obtained at stage b, of a multilayer coating, which is a composite with a thickness of up to 2 microns, performed in two stages: sputtering a titanium cathode and pulse-arc sputtering of calcium hydroxyapatite and a graphite cathode with a gradual increase in carbon concentration from 10 to 55 wt%; - Stage 2, includes the deposition of a carbon nano-coating up to 1.0 µm thick over the first composite layer with a hardness of 100-120 GPa by pulse-arc sputtering of a graphite cathode under conditions of condensation of a diamond-like film at a temperature not exceeding 150 ° C and an energy of carbon ions not exceeding 100 eV.

In particular embodiments of the invention, the bulk nanostructured titanium is VT5-0 titanium.

In particular embodiments of the invention, the surface of the implant obtained in stage a is characterized by a microhardness of 3000-3500 MPa, a tensile strength of 850-1200 MPa and a yield point of 800-1100 MPa.

In particular embodiments of the invention, the implant is 3D printed using the DMLS direct metal laser sintering technology.

The technical result is also achieved by creating a titanium implant with a bioactive nanocoating based on a calcium-phosphate-carbon composite by the above method.

### Definitions and terms

For a better understanding of the present invention, below are some of the terms used in the present description of the invention.

In the description of this invention, the terms **"include"** and **"including"** are interpreted to mean "includes, among other things". These terms are not intended to be construed as "consists only of'.

### Detailed disclosure of the invention

Implant printing on a 3D printer using direct laser metal sintering (DMLS) technology from VT5-0 titanium with a characteristic grain size of no more than 0.01 mm, allows you to create the required microrelief of the implant surface with a high degree of porosity, and the required physical properties of the surface with microhardness 3000-3500 MPa, tensile strength 850-1200 MPa and yield strength 800-1100 MPa. The microrelief of the implant surface with pores provides an increased level of adhesion of the applied composite coating. Plasma treatment of the implant surface with argon at a pressure of (2-6) × 10-2 Pa under a negative voltage with a gradual increase from 800 to 1500 V for 1 hour ensures that the surface is cleaned from contaminants by removing the surface layer.

The composite coating layer is carried out in a two-stage sputtering process. The first stage is a layer of a composite consisting of titanium compounds with carbon and calcium phosphate (calcium hydroxyapatite) is applied by simultaneous arc sputtering of a titanium cathode and pulse-arc sputtering of calcium hydroxyapatite and a graphite cathode with an increase in carbon concentration from 10 to 55 wt%. The second stage is the deposition of a carbon nanocoating over the first calcium-phosphate-carbon composite layer with a hardness of 100-120 GPa by pulse-arc sputtering of a graphite cathode during the condensation of a diamond-like film at a temperature of no more than 150 ° C and an energy of carbon ions no more than 100 eV, which allows to obtain a nanostructured coating several microns thick, chemically resistant to the environment. As a result of coating, corrosion of titanium in the body decreased, the negative effect of titanium ions on the viability of osteoblasts, osteoclasts, epithelial cells decreased, in contrast to uncoated implants. The coating does not dissolve under the influence of the environment of a living organism, since the thickness of the coating before introduction into the body and after its extraction was the same.

Chemical resistance is achieved by reducing the likelihood of cracking in the coating due to the uniformity of the coating structure.

An increase in the carbon concentration from 10 to 55 wt% with increasing coating thickness provides sufficient hardness and mechanical strength of the composite coating. The low carbon concentration and the predominance of titanium and calcium hydroxyapatite at the point of direct contact of the coating with the implant surface increases the adhesion of the coating.

The increased content of calcium hydroxyapatite in the middle of the composite coating contributes to the formation of a coating structure similar to natural bone tissue, since calcium hydroxyapatite is the main inorganic component of bone tissue. This increases the osseointegration of the implants. A layer of calcium hydroxyapatite allows you to create a special coating structure with physicochemical characteristics. A network of truncated pyramid-shaped elevations is formed on the surface of the pavement due to the pavement structure. Their height can reach 300 nm. It was found that on a surface with a dimension of more than 50 nm and a certain form of porosity, the cells of the osteoblastic differon are deformed. As a result, mechanical signals enter cells and can activate signaling pathways for the start of osteogenesis.

Carbon nanocoating with a thickness of not more than 1.0 µm, deposited on a calcium-phosphate-carbon composite layer by pulse-arc sputtering of a graphite cathode during condensation of a diamond-like film at a temperature not exceeding 150 ° C and an energy of carbon ions not exceeding 100 eV, increases the mechanical strength of the implant surface, its biocompatibility and bactericidal properties. The resulting solid calcium-phosphate-carbon composite layer is biologically compatible with the tissues of living objects, non-toxic, has the property of osteogenesis.

### Examples of the invention

The possibility of objectively achieving the technical result in the implementation of the invention is confirmed by reliable data given in the examples containing experimental information. It should be understood that these and all examples given in the application materials are not limiting and are given only to illustrate the present invention.

The method according to the invention is carried out as follows. The implant is 3D printed using direct metal laser sintering (DMLS) technology from titanium. The process involves using a 3D STL model of the implant as blueprints to build a physical object. The implant model is digitized and virtually divided into thin layers with a thickness of up to 20 micron. The finished "construction" file is used as a set of drawings during printing. As a heating element for sintering metal powder, fiber-optic lasers of relatively high power - about 200 W. Powder material, in particular, titanium grade VT5-0 with a characteristic grain size of no more than 0.1 microns is fed into the working chamber in quantities required for the application of one layer. The quantity is determined automatically by the printer itself. A special roller evens the supplied material into an even layer and removes excess material from the chamber, after which the laser head sinters the fresh powder particles between themselves and with the previous layer according to the contours defined by the digital model. After finishing drawing the layer, the process is repeated: the roller delivers fresh material and the laser begins to sinter the next layer. This technology makes it possible to create the required microrelief of the implant surface with a high degree of porosity, and the required physical properties of the surface with a microhardness of 3000-3500 MPa, tensile strength of 850-1200 MPa and yield strength of 800-1100 MPa. The microrelief of the implant surface with pores provides an increased level of adhesion of the applied composite coating.

The printed implant on special holders is placed in the chamber of the sputtering unit and evacuated to a pressure of 10-4 Pa.

Plasma treatment of the implant surface with argon at a pressure of (2-6) × 10-2 Pa under a negative voltage with a gradual increase from 800 to 1500 V for 1 hour ensures that the surface is cleaned from contaminants by removing the surface layer.

After argon treatment, the composite layer is applied in a two-stage sputtering process. The layer has a homogeneous structure and consists of compounds of calcium hydroxyapatite with carbon and titanium. The concentration of compounds in the coating varies depending on their depth from the implant surface. A layer of a compound of calcium hydroxyapatite with carbon and titanium is applied by simultaneous arc sputtering of a titanium cathode and pulse-arc sputtering of calcium hydroxyapatite and a graphite cathode with an increase in the frequency of carbon pulses (Table 1).

**Table 1. Concentration of substances in the composite nanocoating.**

| Time, hour | Frequency Hz | C, weight, % | Ti, weight, % | Ca-P (hydroxyapatite), weight, % |
|---|---|---|---|---|
| 1 | 1 | 10,6 | 78,9 | 10,5 |
| 2 | 5 | 21,1 | 11,8 | 67,1 |
| 3 | 10 | 33,4 | 11,2 | 55,4 |
| 4 | 15 | 46,7 | 16,6 | 36,7 |
| 5 | 20 | 54,9 | 12,8 | 32,3 |

This mode of operation of the carbon source provides an increase in the concentration of carbon in the layers of compounds of calcium hydroxyapatite and titanium with carbon from 10.6 to 55 wt.%.

At the second stage, a fixing layer of solid amorphous diamond-like carbon with a hardness of 100-120 GPa is sprayed by pulse-arc sputtering of a graphite cathode. The structure of the composite coating has a homogeneous structure that blocks crack propagation, providing a higher mechanical and chemical resistance of the coating, which is important for dental implants operating under stress and in an aggressive environment (saliva, food additives, etc.).

Using the sputtering of a graphite target by the pulse-arc method, a diamond-like carbon nanocoating is applied at a temperature on the substrate not exceeding 150 ° C and an ion energy not exceeding 100 eV. The thickness of the diamond-like coating is no more than 1 micron.

### Studies of implants obtained by the method according to the invention in various in vitro and in vivo experiments

The studies were carried out on 60 rabbits. An in vitro experiment was performed using 10 sexually mature herd-bred rabbits, in vivo on 50 rabbits. All manipulations with animals were performed in accordance with the requirements of GOST R ISO 10993.2-99.

In vitro experiments revealed the absence of the toxic effect of the composite nanocoating of the implants according to the invention (NC) on rabbit myelokaryocytes, through assessing the colony-forming ability and viability of myelokaryocytes cultured in the presence of different groups of implants, for example, implants with mechanical surface treatment M-Ti (titanium implants without additional surface treatment made on a CNC lathe (CNC-numerical control), group of implants SLA (implants with a surface subjected to coarse sandblasting and acid etched), implants with a surface SLA + Nano / CaP (SLA coated with Nano / CaP (hydroxyapatite)), group Nano-C (implant with carbon diamond-like coating obtained by the method according to RU2571559) and NC implants according to the invention (the surface of the implants was modified with a composite coating with the addition of calcium and carbon hydroxyapatite, obtained by the pulsed arc method sputtering of hydroxyapatite and a graphite target according to the invention). The comparison implants were manufactured on CNC lathes from grade 5 titanium. The implants according to the invention are NC 3D printed titanium grade 5 titanium. Implant diameter 3.5 mm, length 5 mm. A total of 192 implants were prepared.

The cytotoxicity of the implants was assessed by the activity of lactate dehydrogenase (LDH) in myelokaryocyte supernatant after incubation of rabbit myelokaryocytes with M-Ti, SLA, SLA + Nano / CaP, Nano-C and NC implant samples. The cultivation of myelokaryocytes was carried out under standard conditions: 2 types of complete culture medium were used: for the cultivation of the adherent fraction of myelokaryocytes (type I) - RPMI-1640 (FGU SSC "Vector"), 20% fetal calf serum (HyClone) L-glutamine 30 mg / 100 ml medium (Sigma), gentamicin - 5 mg / 100 ml of medium, seeding density 5-6x106 / ml of living cells. For functional assessment of hematopoietic cells (type II) - semi-liquid synthetic ready-made MethoCult ™ medium containing erythropoietin, recombinant human cytokines - stem cell factor (rhSCF), colony-stimulating factors - granulocyte-macrophage (rhGM-CSF), granulocyte-CSF (rhG) interleukins - IL-3, -6 (rhIL-3, rhIL-6), seeding density 1x104 / ml of live bone marrow mononuclear cells. The cultivation was carried out in a CELL 48 CO2 incubator for 3-32 days, at an absolute humidity, 37oC, 4% CO2.

In vitro studies have shown that cell viability upon incubation of myelokaryocytes with NC implant samples tended to increase compared to other implants. The morphology of adherent rabbit myelokaryocytes, in wells without samples or with M-Ti, SLA, SLA + Nano / CaP, Nano-C, as well as in the presence of NC, did not differ: most of the cells had a typical fibroblast-like morphology. Closely packed parallel fusiform cells predominated. Cultures did not stop growth upon reaching a confluent monolayer, forming multilayer cords. During cultivation on porous titanium samples on day 3, hematopoietic cells and stromal elements were mainly revealed in the pores of titanium matrices, some of them were located in morphologically homogeneous groups of cells - erythroid and granulocyte-macrophage, which suggests their formation by differentiation of CFUe and CFUgm. By the method of scanning electron microscopy, the emerging extracellular substance was revealed; by the 17th to 21st days, part of the pores was completely filled with the components of the extracellular matrix. On the 32nd day, the number of cells in the surface pores during the cultivation of myelokaryocytes on NC implants was significantly higher compared to other implants, namely by 266% (80; 213 - the number of osteogenic cells per 100 µm2) higher than on M-Ti implants, 208% (102; 213) higher than on SLA implants, 163% (130, 213) higher than on SLA + Nano / CaP implants, and 141% (151, 213) higher than on Nano implants -C.

Thus, NC implants had a more stable tendency to increase the rates of osteogenesis, which contributed to the formation of stronger and more viable living tissue around the implant. In comparison with other implants, reliable fixation of the implant at an early date and more durable secondary fixation at a later date. What is important for fast and quality treatment. In in vivo experiments, cylindrical implants M-Ti, SLA, SLA + Nano / CaP, Nano-C, NC, pre-saturated with autologous myelokaryocytes, increased in number by culturing for 14 days, were implanted into the tibia and femur in rabbits. The controls were M-Ti implants saturated with autologous myelokaryocytes, increased in number by cultivation for 14 days, and intact rabbits. The timing of the withdrawal of animals from the experiment is 2, 4, 16, 52 weeks after the operation.

In an in vivo experiment, the morphology of bone tissue was studied, and its mechanical tests were carried out. The tensile strength of the bone tissue at the interface "bone bed - implant" was studied according to the original method on a universal testing machine FP 100/1 and was expressed as a% of the strength of intact bone tissue. To study the newly formed bone tissue in the pore space, the titanium matrix was etched by deep etching according to Mirgazizov. Bone tissue was examined by scanning electron microscopy (on a QUANTA 200 microscope) or by light microscopy on thin sections after decalcification (in BiodecR solution), dehydration in alcohol, and staining with hematoxylin and eosin. To objectify the results, the area of the mature bone tissue newly formed in the pores of the implant was measured using the hardware-software complex VideoTesT-Master "Morphology" 4.0. The number of osteogenic cells per 100 µm2 of the histological preparation was counted, the alkaline phosphatase (ALP) activity in the cells of the osteocytic lineage was histochemically detected using a Bio-Optica reagent kit, and the volume fraction of the profiles of the osteocytic lineage cells with ALP activity was determined. Images were entered using a digital VIDI-CAM module at a magnification of 400. Image analysis was performed using the VideoTesT Master-Morphology 5.2 software.

In peripheral blood, the concentration of bone morphogenetic protein-2 (BMP-2) and insulin-like growth factor-1 (IGF-1) was determined by the enzyme-linked immunosorbent assay using the Cloud-Clone Corp Organism Species: Oryctolagus cuniculus (Rabbit) kits according to the protocols supplied with the kits using controls. one). The results are described using statistical characteristics - median (Me) and quartiles (Q25; Q75). To identify differences between the two groups of quantitative traits, nonparametric criteria were used for dependent (Wilcoxon test) and independent (Mann-Whitney test) groups using multiple hypothesis testing technologies based on the Bonferroni correction. The statistical hypothesis was considered confirmed at a significance level of p≤0.05.

In in vivo studies, the implementation of the NC implants according to the invention demonstrated a higher and more long-term activity of osteogenesis processes in comparison with M-Ti, SLA, SLA + Nano / CaP, Nano-C implants. When the NC implants were inserted 2 weeks after the operation, a significant increase in the number of osteogenic cells, cells expressing alkaline phosphatase was revealed, the volume fraction of the profiles of osteocytic cells expressing alkaline phosphatase increased in the segment of bone tissue adjacent to the defect as compared to their representation in intact bone tissue and in animal bone tissue, with embedded M-Ti, SLA, SLA + Nano / CaP, Nano-C implants. As a result, the area of the internal pore space occupied by the mature bone tissue, during the introduction of NC implants, exceeded the area of the mature bone tissue after the introduction of M-Ti, SLA, SLA + Nano / CaP, Nano-C implants and was 67% after 2 weeks, after 52 weeks - 98%; compared to M-Ti (24% and 39%), with SLA (32% and 54%), with SLA + Nano / CaP (42% and 68%), Nano-C (42% and 69%), respectively. The tensile strength of bone tissue at the interface "bone bed - implant" was also significantly higher after implantation of NC implants compared to M-Ti at early follow-up: after 4 weeks - 58%, 16 weeks - 81%; with M-Ti implantation after 4 weeks - 20.5%, 16 weeks - 27%; with SLA implantation after 4 weeks - 35%, 16 weeks - 47%, with SLA + Nano / CaP implantation after 4 weeks - 40%, 16 weeks - 51%, with Nano-C implantation after 4 weeks - 50%, 16 weeks - 71% respectively. When using NC implants, in comparison with other implants, a better condition of the bone bed was noted in the early stages, characterized by less severity of dystrophic, necrotic processes, rarefaction of the adjacent bone tissue, and in the later stages (52 weeks after surgery) - less pronounced sclerotic changes in the newly formed bone tissue.

The mechanisms of regulation of reparative regeneration of bone tissue are mediated by systemic (hormonal, neuroendocrine factors, vitamins) and local (growth factors, cytokines, bone morphogenetic proteins) factors.

**Table 2. Concentration of growth factors**

| Implant type | Concentration of KMB-2 (local growth factor) | | IGF-1 concentration | |
|---|---|---|---|---|
| | 4 weeks | 16 weeks | 4 weeks | 16 weeks |
| M-Ti (control) | 194,1% | 0% | hasn't changed | hasn't changed |
| SLA | 216% | 15% | 94% | 5% |
| SLA + Nano / CaP | 250% | 50% | 150% | 35% |
| Nano-C | 261% | 55% | 160% | 43% |
| NC | 485% | 150% | 399% | 98% |

As can be seen from the table, the concentration of IGF-1 in peripheral blood in rabbits with NC implants was increased within 16 weeks after surgery with a maximum change after 4 weeks - 399% (p≤0.017), compared to the level before surgery. The dynamics of the concentration of KMB-2 is similar. 4 weeks after surgery, it increases to 485% (p≤0.017) compared to the level before surgery and significantly exceeds the same indicator in other groups by more than 2 times. The participation of systemic regulators of osteogenesis, a more pronounced increase in the concentration of local factors inducing osteogenesis, indirectly confirms the greater intensity of the processes of reparative osteogenesis.

Thus, in vitro studies with the implant obtained by the method according to the invention revealed a greater viability of myelokaryocytes, a tendency to increase the colony-forming ability of the adherent fraction of myelokaryocytes when using the NC implants according to the invention in comparison with other implants.

In in vivo studies, the use of the NC implants of the invention leads to the formation of more mature and stronger bone tissue in the pores of the implants, compared to other comparative implants.

In addition, the NC implants according to the invention are characterized by bactericidal properties, slowing down the processes of hardening of newly formed bone tissue.

Thus, NC implants have the ability to activate osteogenesis at a faster rate. The level of biocompatibility of NC dental implants with composite nanocoating according to the invention is higher than that of existing analogues.

### Clinical examples of the use of implants according to the invention

**Example 1.** Patient male - age 59 years. Secondary adentia of the lower jaw. Partial adentia of the upper jaw. Installed are titanium implants printed on a 3D printer with a composite nano-coating applied according to the invention. During the installation of the intraosseous implant, an implant physiological dispenser was used. Installation of implants was carried out in the mode of 30 revolutions and with a load of 30 N. The implant was placed in the implant bed without additional fixation with artificial bone.

Result:
1. After two weeks, stability was noted, good secondary fixation of the implant in the implant bed was observed.
2. After two months, the condition of the implant is stable. The plugs were replaced with abutments with ball-shaped attachments (locks) that fix removable dentures.
3. After ten months of observation, the condition of the implants is stable. The newly formed bone tissue has completely grown into the body of the implant and filled the inter-root space.

**Example 2.** Patient woman - age 68 years. According to the patient, he is in a state after coronary bypass surgery and is taking blood-thinning drugs. Implants made of titanium alloy with composite nanocoating were installed. The implants were installed in the same modes as in the first example.

Result:
1. After two weeks, stability was noted, the control period for the growth of the trabecular bone into the implant body favorably changed the primary fixation.
2. After two months - the implants are stable. The plugs were replaced with abutments and a bridge prosthetics with a plastic construction with implant fixation was performed.
3. After ten months of observation, the condition of the implants is stable. The interroot space is filled with new bone tissue.

**Example 3.** Patient male - age 47 years. Suffers from grade 2 diabetes. One cyst in area 3.6. Removal, cystectomy through the lateral approach and simultaneous installation of a titanium alloy implant with composite nanocoating into the preserved interroot septum were performed.

Result:
1. After two weeks, stability was noted, good secondary fixation of the implant was observed, and bone growth to the body of the implant was observed.
2. After two months - the implants are stable. The plugs were replaced with abutments with subsequent prosthetics of the metal-ceramic crown.
3. After ten months - the condition of the implants is stable. The new bone tissue has completely filled the interroot septum and has grown to the very body of the implant.

As a result of the tests carried out, it was found that there were no signs of rejection, inflammation, suppuration, allergic reactions in patients with installed implants. In all cases, the new bone tissue completely filled all the free inter-root space in the implant bed and adhered to the implant body, which ensures incredible stability of the implant at the level of the natural tooth. This leads to the absolute minimization of the risk of losing the implant due to natural causes. This result has not been achieved with other implants. The invention can be used in dental implantology in outpatient dental facilities.

Although the invention has been described with reference to the disclosed embodiments, it should be apparent to those skilled in the art that the specific experiments described in detail are provided for the purpose of illustrating the present invention only and should not be construed as limiting the scope of the invention in any way. It should be understood that various modifications are possible without departing from the spirit of the present invention.

## Claims

1. A method of obtaining a dental implant with a bioactive nanocoating based on a calcium-phosphate-carbon composite, including the following steps:
a) printing the implant on a titanium 3D printer;
b) treatment of the surface of the implant obtained at stage a, including the treatment of the implant with argon ions accelerated to 1 keV at a pressure of (2-6) × 10-2 Pa with the application of a negative bias voltage with a gradual increase from 800 to 1500 V for 1 h;
c) layer-by-layer deposition on the implant base, obtained at stage b, of a multilayer coating, which is a composite with a thickness of up to 2 µm, performed in two stages:
- Stage 1, includes the deposition of a composite layer consisting of a titanium-carbon compound and calcium hydroxyapatite, simultaneous arc spraying of a titanium cathode and pulse-arc sputtering of calcium hydroxyapatite and a graphite cathode with a gradual increase in carbon concentration from 10 to 55 wt%; - Stage 2, includes the deposition of a carbon nano-coating up to 1.0 µm thick over the first composite layer with a hardness of 100-120 GPa by pulse-arc sputtering of a graphite cathode under conditions of condensation of a diamond-like film at a temperature not exceeding 150 ° C and an energy of carbon ions not exceeding 100 eV.

2. The method of claim 1, wherein the titanium is VT5-0 titanium.

3. The method according to claim 1, wherein the surface of the implant obtained in stage a is **characterized by** a microhardness of 3000-3500 MPa, a tensile strength of 850-1200 MPa and a yield point of 800-1100 MPa.

4. The method of claim 1, wherein the 3D printing of the implant is carried out using the DMLS direct metal laser sintering technology.

5. A titanium implant with a bioactive nanocoating based on a calcium-phosphate-carbon composite obtained by the method of claim 1.
